(19) Europäisches Patentamt
European Patent Office
Office européen des brevets

(11) Veröffentlichungsnummer: **0 313 983 A2**

(12) # EUROPÄISCHE PATENTANMELDUNG

(21) Anmeldenummer: 88117453.6

(22) Anmeldetag: 20.10.88

(51) Int. Cl.⁴: C07D 249/08 , A61K 31/41

(30) Priorität: 30.10.87 DE 3736747

(43) Veröffentlichungstag der Anmeldung: 03.05.89 Patentblatt 89/18

(84) Benannte Vertragsstaaten: AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Krämer, Wolfgang, Dr.
Rosenkranz 25
D-5093 Burscheid 2(DE)
Erfinder: Holmwood, Graham, Dr.
Krutscheider Weg 105
D-5600 Wuppertal 11(DE)
Erfinder: Büchel, Karl Heinz, Prof. Dr.
Dabringhauser Strasse 42
D-5093 Burscheid(DE)
Erfinder: Plempel, Manfred, Dr.
Zwengenbergerstrasse 3 c
D-5657 Haan(DE)

(54) **Verwendung neuer Triazolylalkanole zur Behandlung von Krankheiten.**

(57) Die Erfindung betrifft die Verwendung neuer substituierter Triazolylalkanole der Formel

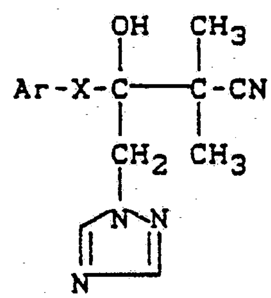

zur Behandlung von Krankheiten, insbesondere Mykosen.

EP 0 313 983 A2

## Verwendung neuer Triazolylalkanole zur Behandlung von Krankheiten

Die Erfindung betrifft die Verwendung von neuen substituierten Triazolylalkanolen zur Behandlung von Krankheiten, insbesondere von Mykosen.

Es ist bekannt, daß bestimmte substituierte Triazolylalkanole, wie beispielsweise das 3,3-Dimethyl-4-fluor-1-(4-methylphenoxy)-2-(1,2,4-triazol-1-ylmethyl)-butan-2-ol oder das 1-(4-Chlor-2-methylphenoxy)-3,3-dimethyl-4-fluor-2-(1,2,4-triazol-1-ylmethyl)-butan-2-ol antimykotische Eigenschaften besitzen (vergl. DE-OS 32 02 613).

Die Wirksamkeit dieser vorbekannten Verbindungen ist jedoch nicht in allen Indikationen völlig zufriedenstellend.

Es wurde gefunden, daß die neuen substituierten Triazolylalkanole der allgemeinen Formel (I),

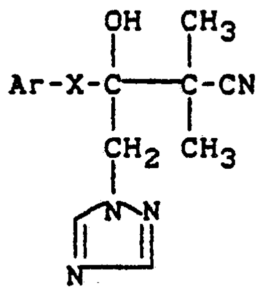

(I)

in welcher

Ar für gegebenenfalls substituiertes Aryl steht und

X für eine der Gruppen $-CH_2-$; $-O-CH_2-$; $-S-CH_2-$; $-CH_2-CH_2-$; $-CH=CH-$ oder $-C\equiv C-$ steht,

sowie deren physiologisch verträgliche Säureadditionssalze gute antimikrobielle, insbesondere gute antimykotische Eigenschaften besitzen.

Die Verbindungen der Formel (I) können als optische Isomere oder Isomerengemische unterschiedlicher Zusammensetzung vorliegen. Sowohl die Verwendung der reinen Isomeren als auch die der Isomerengemische werden erfindungsgemäß beansprucht.

Überraschenderweise zeigen die neuen, erfindungsgemäß verwendbaren substituierten Triazolylalkanole der allgemeinen Formel (I) in bestimmten Indikationen bei vergleichbar guter in-vitro-Wirksamkeit eine deutlich bessere in-vivo-Wirksamkeit als die aus dem Stand der Technik bekannten substituierten Triazolylalkanole, wie beispielsweise das 3,3-Dimethyl-4-fluor-1-(4-methyl phenoxy)-2-(1,2,4-triazol-1-ylmethyl)-butan-2-ol oder das 1-(4-Chlor-2-methylphenoxy)-3,3-dimethyl-4-fluor-2-(1,2,4-triazol-1-ylmethyl)-butan-2-ol, welches strukturell ähnliche Verbindungen sind.

Die erfindungsgemäß verwendbaren substituierten Triazolylalkanole sind durch die Formel (I) allgemein definiert. Bevorzugt erfindungsgemäß verwendbar sind Verbindungen der Formel (I), bei welchen

Ar für gegebenenfalls einfach oder mehrfach, gleich oder verschieden substituiertes Phenyl steht, wobei als Substituenten infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor und Brom sowie jeweils gegebenenfalls einfach oder mehrfach, gleich oder verschieden durch Halogen substituiertes Phenyl oder Phenoxy und

X für eine der Gruppen $-CH_2-$; $-O-CH_2-$; $-S-CH_2-$; $-CH_2-CH_2-$; $-CH=CH-$ oder $-C\equiv C-$ steht.

Ganz besonders bevorzugt verwendbar sind Verbindungen der Formel (I), bei welchen

Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio sowie jeweils gegebenenfalls ein-bis dreifach, gleich oder verschieden durch Fluor, Chlor und/oder Brom substituiertes Phenyl oder Phenoxy und

X für eine der Gruppen $-CH_2-$; $-O-CH_2-$; $-S-CH_2-$; $-CH_2-CH_2-$; $-CH=CH-$ oder $-C\equiv C-$ steht.

Bevorzugt erfindungsgemäß verwendbare Verbindungen sind auch Additionsprodukte aus Säuren und denjenigen substituierten Triazolylalkanolen der Formel (I), in denen die Substituenten Ar und X die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesonder Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Im einzelnen seien außer den bei den Herstellungsbeispielen genannten Verbindungen die folgenden substituierten Triazolylalkanole der allgemeinen Formel (I) genannt:

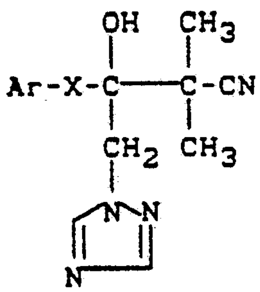

(I)

| Ar | X | Ar | X |
|---|---|---|---|
| 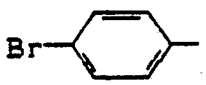 | $-OCH_2-$ | 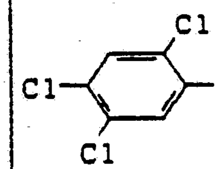 | $-CH_2-CH_2-$ |
| 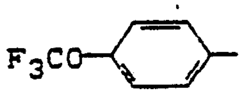 | $-CH=CH-$ | 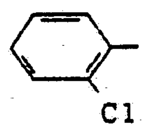 | $-CH_2-CH_2-$ |
| 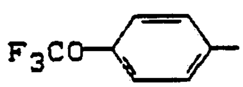 | $-CH_2-CH_2-$ | 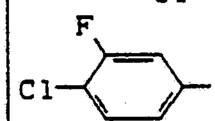 | $-CH_2-CH_2-$ |
| 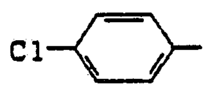 | $-C\equiv C-$ | 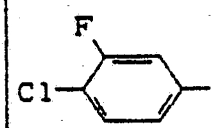 | $-CH=CH-$ |
| 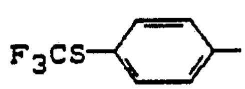 | $-O-CH_2-$ | 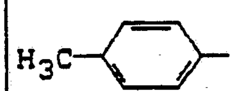 | $-CH_2-$ |
| 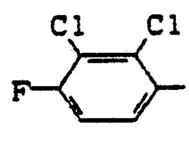 | $-OCH_2-$ | 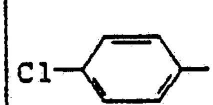 | $-CH_2-$ |
| 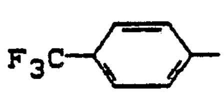 | $-CH_2-CH_2-$ | | |

| Ar | X | Ar | X |
|---|---|---|---|
| 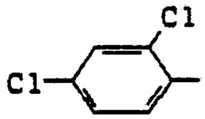 | -CH2- | 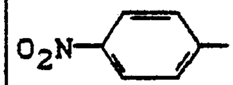 | $-CH_2-$ |
| 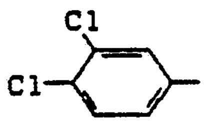 | $-CH_2-$ | 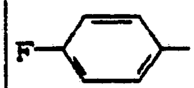 | $-CH_2-$ |
| 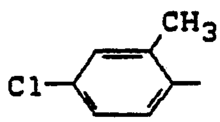 | $-O-CH_2-$ | | |

Die erfindungsgemäß verwendbaren substituierten Triazolylalkanole der Formel (I) sowie deren Säureadditionssalze sind noch nicht bekannt. Sie sind jedoch Gegenstand einer eigenen parallelen Patentanmeldung und können nach dem dort beschriebenen Verfahren erhalten werden, indem man Oxime der Formel (II),

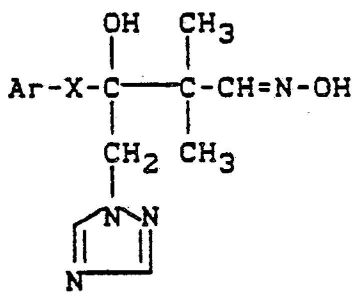

(II)

in welcher
Ar und X die oben angegebene Bedeutung haben,
mit einem wasserabspaltenden Mittel, gegebenenfalls in Gegenwart eines Verdünnungsmittels und gegebenenfalls in Gegenwart eines Reaktionshilfsmittels, umsetzt und gegebenenfalls anschließend eine Säure oder ein Metallsalz addiert.

Verwendet man beispielsweise 2,2-Dimethyl-3-(4-chlor-2-fluorbenzyl)-3-hydroxy-4-(1,2,4-triazol-1-yl)-butanaloxim als Ausgangsverbindung und Acetanhydrid als wasserabspaltendes Mittel, so läßt sich der Reaktionsablauf des Herstellungsverfahrens durch das folgende Formelschema darstellen:

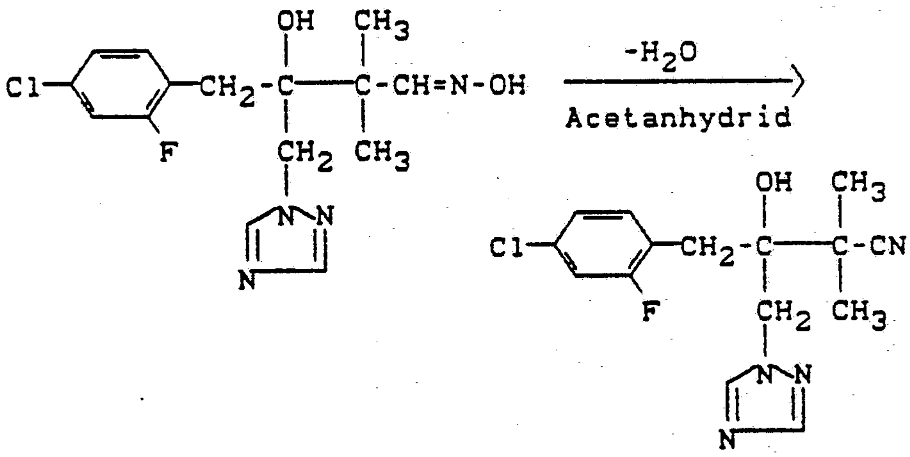


Die zur Durchführung des Herstellungsverfahrens als Ausgansstoffe benötigten Oxime sind durch die Formel (II) allgemein definiert. In dieser Formel (II) stehen Ar und X vorzugsweise für diejenigen Reste, die bereits im Zusammenhang mit der Beschreibung der erfindungsgemäß verwendbaren Stoffe der Formel (I) als bevorzugt für diese Substituenten genannt wurden.

Die Oxime der Formel (II) sind bekannt oder können in Analogie zu bekannten Verfahren erhalten werden (vergl. DE-OS 33 34 779 sowie die Herstellungsbeispiele).

Das Herstellungsverfahren wird in Gegenwart eines geeigneten wasserabspaltenden Mittels durchgeführt. Als solche kommen alle üblichen Dehydratisierungsmittel infrage (vergl. hierzu beispielsweise C.Ferri "Reaktionen der organsichen Synthese", S.572; Thieme Verlag, Stuttgart 1978). Mit besonderem Vorzug verwendet man Carbonsäureanhydride, wie beispielsweise Acetanhydrid.

Als Verdünnungsmittel zur Durchführung des Herstellungsverfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Ester, wie Essigsäureethylester.

Es ist auch möglich, bei Verwendung flüssiger, wasserabspaltender Mittel, diese in einem entsprechenden Überschuß gleichzeitig als Verdünnungsmittel einzusetzen.

Das Herstellungsverfahren wird gegebenenfalls in Gegenwart eines geeigneten Reaktionshilfsmittels durchgeführt. Als solche kommen anorganische oder organische Basen in Frage. Hierzu gehören beispielsweise Alkalimetallcarbonate oder -acetate, wie Natriumcarbonat, Natriumacetat, Kaliumcarbonat oder Natriumhydrogencarbonat, sowie tertiäre Amine, wie Triethylamin, N,N-Dimethylanilin, Pyridin oder N,N-Dimethylaminopyridin.

Es ist jedoch auch möglich, das Herstellungsverfahren ohne Zusatz eines Reaktionshilfsmittels durchzuführen.

Die Reaktionstemperaturen können bei der Durchführung des Herstellungsverfahrens in einem größeren Bereich variiert werden. Im allgemeinen arbeitet man bei Temperaturen zwischen 0° C und 120° C, vorzugsweise bei Temperaturen zwischen 40° C und 110° C.

Zur Durchführung des Herstellungsverfahrens setzt man pro Mol an Oxim der Formel (II) im allgemeinen 1.0 bis 50.0 Mol, vorzugsweise 1.0 bis 10.0 Mol an wasserabspaltendem Mittel und gegebenenfalls 1.0 bis 5.0 Mol, vorzugsweise 1.0 bis 2.5 Mol an Reaktionshilfsmittel ein. Die Reaktionsdurchführung, Aufarbeitung und Isolierung der Reaktionsprodukte erfolgt nach allgemein üblichen Methoden.

Zur Herstellung von physiologisch verträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen

Lösungsmittel gereinigt werden.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid,

Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele:

Beispiel 1:

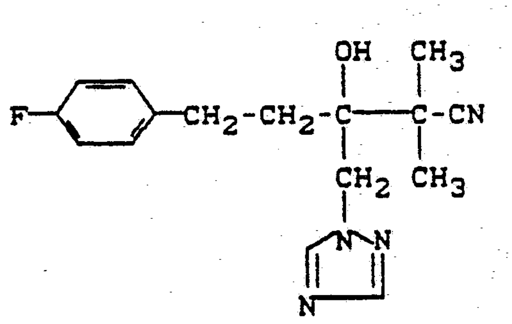

16 g (0,05 Mol) 2,2-Dimethyl-5-(4-fluorphenyl)-3-hydroxy-3-(1,2,4-triazol-1-ylmethyl)-pentanal-oxim werden in 150 ml Acetanhydrid 3 Stunden auf Rückflußtemperatur erhitzt, abgekühlt, auf 200 g Eis gegossen, mit 300 ml Dichlormethan extrahiert, über Natriumsulfat getrocknet und im Vakuum eingeengt. Der Rückstand wird durch Verrühren mit 150 ml Diethylether zur Kristallisation gebracht, abgesaugt und getrocknet.

Man erhält 11 g (73 % der Theorie) an 5-(4-Fluorphenyl)-3-hydroxy-2-methyl-3-(1,2,4-triazol-1-ylmethyl)-pentan-2-carbonitril vom Schmelzpunkt 152-154 °C.

Herstellung der Ausgangsverbindung:

Beispiel II-1

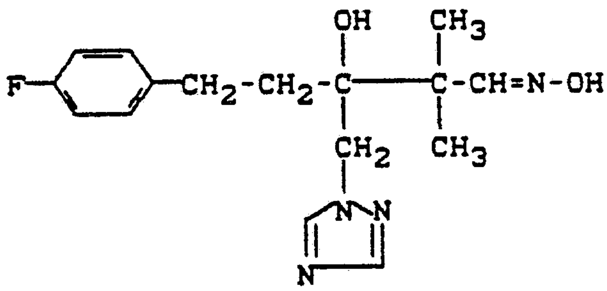

41,2 g (0,135 Mol) 2,2-Dimethyl-5-(4-fluorphenyl)-3-hydroxy-3-(1,2,4-triazol-1-ylmethyl)-pentanal und 11,1 g (0,16 Mol) Hydroxylaminhydrochlorid werden in 300 ml Ethanol 6 Stunden unter Rückfluß erhitzt, abgekühlt, in 1000 ml gesättigte wässrige. Natriumhydrogencarbonatlösung gegeben und zweimal mit jeweils 300 ml Dichlormethan extrahiert. Die vereinigten organischen Phasen werden zweimal mit jeweils 400 ml Wasser gewaschen, über Natriumsulfat getrocknet und im Vakuum eingeengt. Der Rückstand wird aus Diisopropylether umkristallisiert.

Man erhält 30 g (70 % der Theorie) an 2,2-Dimethyl-5-(4-fluorphenyl)-3-hydroxy-3-(1,2,4-triazol-1-ylmethyl)-pentanal-oxim von Schmelzpunkt 101 °C bis 103 °C.

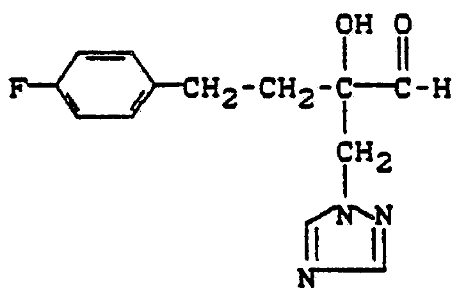

Zu 124 g (0,355 Mol) 2-(1,3-Dioxolan-2-yl)-5-(4-fluorphenyl)-2-methyl-3-(1,2,4-triazol-1-ylmethyl)-pentan-3-ol in einer Mischung aus 850 ml Ethanol und 850 ml Wasser gibt man 85 ml konzentrierte Salzsäure, rührt 16 Stunden bei Raumtemperatur, engt im Wasserstrahlvakuum auf die Hälfte des Volumens ein, neutralisiert mit wässriger Natriumbicarbonatlösung, extrahiert mit 600 ml Dichlormethan, wäscht die vereinigten organischen Phasen mit 1500 ml Wasser, trocknet über Natriumsulfat und engt im Vakuum ein. Der Rückstand wird in 600 ml Aceton aufgenommen, bei 0 °C mit 48 g 1,8-Naphthalindisulfonsäure in 80 ml Aceton versetzt und 4 Stunden bei dieser Temperatur gerührt; der entstandene Niederschlag wird abgesaugt und in 2500 ml gesättigter wässriger Natriumbicarbonatlösung eingerührt. Man extrahiert mehrfach mit insbesamt 600 ml Dichlormethan, trocknet über Natriumsulfat, engt im Vakuum ein und kristallisiert den Rückstand aus Diisopropylether um.

Man erhält 64 g (59 % Theorie) an 2,2-Dimethyl-5-(4-fluorphenyl)-3-hydroxy-3-(1,2,4-triazol-1-ylmethyl)-pentanal vom Schmelzpunkt 98 °C.

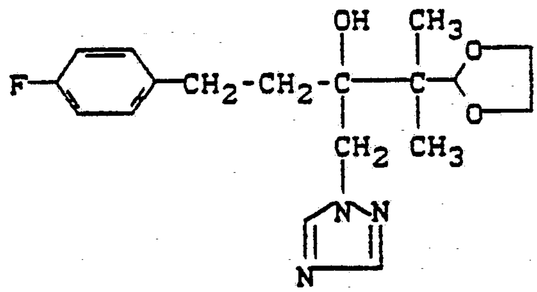

Zu 193 g ( ca. 0,41 Mol) rohem 2-[2-(1,3-Dioxolan-2-yl)-prop-2-yl]-2-[2-(4-fluorphenyl)-ethyl]-oxiran (ca. 60 %ig) in 600 ml n-Butanol gibt man 2,3 g gepulvertes Kaliumhydroxid und erhitzt 16 Stunden auf Rückflußtemperatur. Zur Aufarbeitung engt man im Vakuum ein, nimmt den Rückstand in 700 ml Dichlormethan auf, wäscht zweimal mit jeweils 1500 ml Wasser, trocknet über Natriumsulfat, engt im Vakuum ein und chromatographiert den Rückstand über Kieselgel (Laufmittel: Cyclohexan/Essigester 4:1).

Man erhält 124 g (86 % der Theorie) an 2-(1,3-Dioxolan-2-yl)-5-(4-fluorphenyl)-2-methyl-3-(1,2,4-triazol-1-ylmethyl)-pentan-3-ol als Öl.

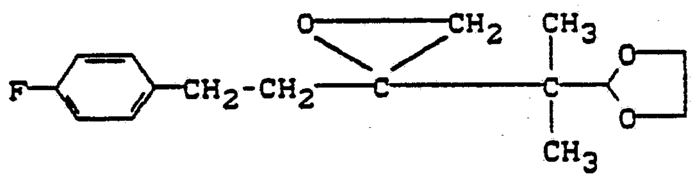

Zu 74,4 g (1,2 Mol) Dimethylsulfid in 350 ml absolutem Dimethylsulfoxid und 200 ml absolutem Tetrahydrofuran tropft man unter Rühren 170,4 g (1,2 Mol) Methyliodid, rührt 16 Stunden bei Raumtemperatur, gibt dann tropfenweise unter Rühren 190 g (0,7 Mol) 2-(1,3-Dioxolan-2-yl)-5-(4-fluorphenyl)-2-methyl-pentan-3-on in 300 ml Toluol zu und dann bei 5 °C portionsweise in einem Zeitraum von 90 Minuten in 3 Portionen 40 g (0,7 Mol) Natriummethylat, rührt weitere 3 Stunden bei Raumtemperatur, gibt dann bei 5 °C innerhalb von 30 Minuten weitere 30 g (0,55 Mol) Natriummethylat in 2 Portionen zu, rührt 10 Stunden bei 20 °C und weitere 8 Stunden bei 45 °C, engt im Vakuum ein, nimmt den Rückstand in 1000 ml Dichlormethan auf, wäscht 3 mal mit jeweils 1000 ml Wasser, trocknet über Natriumsulfat und engt im Vakuum ein.

Man erhält 193 g (ca. 60 % der Theorie) an rohem 2-[2-(1,3-Dioxolan-2-yl)-prop-2-yl]-2-[2-(4-fluorphenyl)ethyl]-oxiran in ca. 60 %iger Reinheit, welches ohne zusätzliche Reinigungsschritte in die nächste Stufe eingesetzt werden kann.

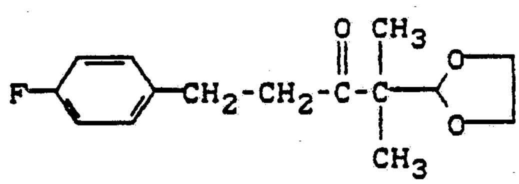

193 g (0,73 Mol) 2-(1,3-Dioxolan-2-yl)-5-(4-fluorphenyl)-2-methyl-pent-4-en-3-on in 1200 ml Tetrahydrofuran werden in Gegenwart von 20 g Raney-Nickel 7 Stunden bei 31 °C und 55 bar Wasserstoffdruck hydriert. Zur Aufarbeitung wird der Katalysator abfiltriert und das Lösungsmittel abdestilliert.

Man erhält 190 g (99 % der Theorie) an 2-(1,3-Dioxolan-2-yl)-5-(4-fluorphenyl)-2-methyl-pentan-3-on als Öl, welches laut gaschromatographischer Analyse in 97 %iger Reinheit vorliegt.

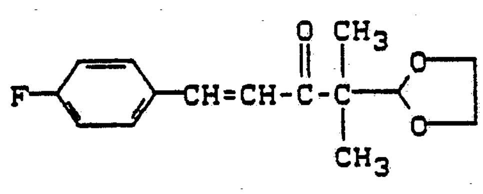

Zu 124 g (1 Mol) 4-Fluorbenzaldehyd und 158 g (1 Mol) 2-(1,3-Dioxolan-2-yl)-2-methyl-butan-3-on (vergl. z.B. DE-OS 32 42 252 oder DE-OS 32 42 236) in 350 ml Ethanol und 124 ml Wasser gibt man tropfenweise unter Rühren 84 ml 10%ige wässrige Natronlauge, rührt nach beendeter Zugabe weitere 16 Stunden bei Raumtemperatur, saugt den ausgefallenen Feststoff ab und wäscht mit wenig Ethanol nach.

Man erhält 206 g (78 % der Theorie) an 2-(1,3-Dioxolan-2-yl)-5-(4-fluorphenyl)-2-methyl-pent-4-en-3-on vom Schmelzpunkt 76 °C, welches aus Petrolether umkristallisiert werden kann.

In entsprechender Weise und gemäß den allgemeinen angaben zur Herstellung erhält man die folgenden substituierten Triazolylalkanole der allgemeinen Formel (I):

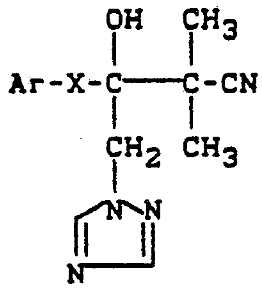

(I)

| Bsp. Nr. | Ar | X | physikalische Konstanten |
|---|---|---|---|
| 2 | 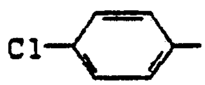 | $-CH_2-CH_2-$ | Fp: 141° C |
| 3 | 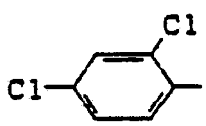 | $-CH_2-CH_2-$ | Fp: 102-104° C |
| 4 | 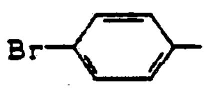 | $-S-CH_2-$ | $^1$H-NMR*):<br>3,24 (q);<br>4,58 (q) |
| 5 | 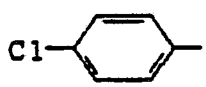 | $-S-CH_2-$ | $^1$H-NMR*):<br>3,23 (q);<br>4,56 (q) |
| 6 | 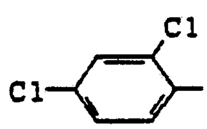 | $-O-CH_2-$ | Fp: 130-132° C |
| 7 | 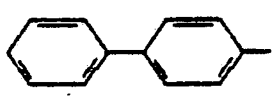 | $-O-CH_2$ | $^1$H-NMR*):<br>3,72 (q);<br>4,71 (q) |
| 8 | 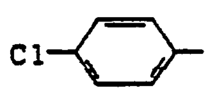 | $-O-CH_2-$ | Fp: 120-122° C |

| Bsp. Nr. | Ar | X | physikalische Konstanten |
|---|---|---|---|
| 9 | 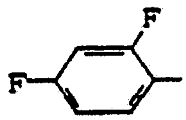 | $-CH_2-CH_2-$ | Fp: 133-135° C |
| 10 | 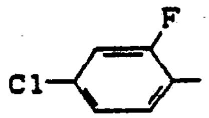 | $-CH_2-CH_2-$ | Fp: 123° C |
| 11 | 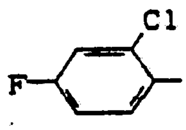 | $-O-CH_2-$ | Fp: 133-135° C |
| 12 | 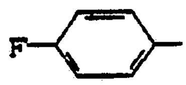 | $-S-CH_2-$ | $^1H$-NMR*): 3,2 (q); 4,55 (q) |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

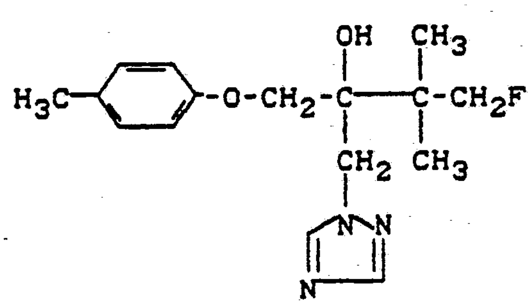 (A)

3,3-Dimethyl-4-fluor-1-(4-methylphenoxy)-2-(1,2,4-triazol-1-ylmethyl)-butan-2-ol (bekannt aus DE-OS 32 02 613)

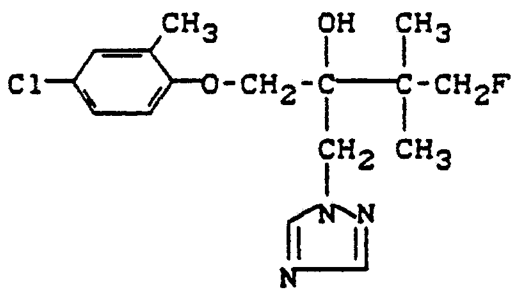

(B)

1-(4-Chlor-2-methylphenoxy)-3,3-dimethyl-4-fluor-2-(1,2,4-triazol-1-ylmethyl)-butan-2-ol (bekannt aus DE-OS 32 02 613)

Beispiel A

Antimykotische in-vitro-Wirksamkeit

Versuchsbeschreibung:

Die in-vitro-Prüfungen wurden im Reihenverdünnungstest mit Keiminokula von durchschnittlich $5 \times 10^3$ bis $10^4$ Keimen/ml Substrat durchgeführt. Als Nährmedium dienten

a) für Dermatophyten und Schimmelpilze:

Sabouraud's milieu d'epreuve

b) für Hefen:

Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigen z. B. die erfindungsgemäß verwendbaren Verbindungen 2, 4, 5, 7 und 10 gute antimykotische Wirksamkeit.

Tabelle A:

Antimykotische in vitro-Wirksamkeit

| Wirkstoff | MHK*)-Werte in µg/ml Nährmedium Tricho-phyton mentagr. | Micro-sporum canis | Candida albicans | Torulopsis glabrata | Aspergillus fumigatus |
|---|---|---|---|---|---|
| (2) | <1 | 16 | 16 | 16 | 16 |
| (4) | <1 | 16 | 8 | 8 | 4 |

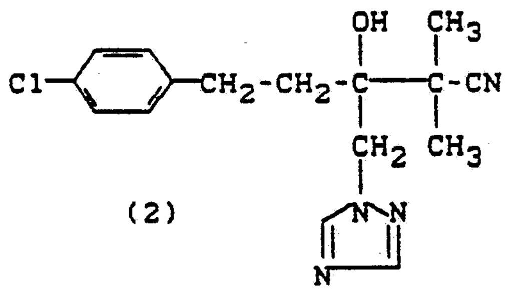

(2)

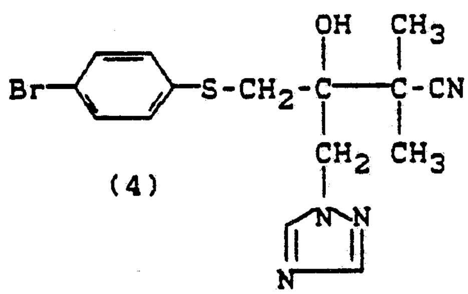

(4)

*)minimale Hemmkonzentration

Tabelle A: (Fortsetzung)

Antimykotische in vitro-Wirksamkeit

| Wirkstoff | MHK*)-Werte in µg/ml Nährmedium Trichophyton mentagr. | Microsporum canis | Candida albicans | Torulopsis glabrata | Aspergillus fumigatus |
|---|---|---|---|---|---|
| (5) | 8 | - | 4 | 4 | 8 |
| 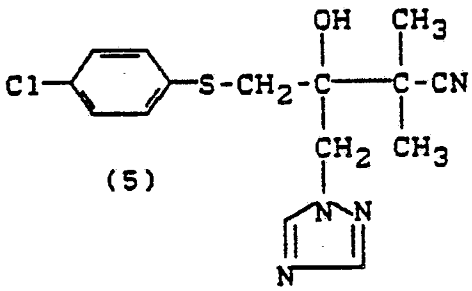 (5) | | | | | |
| (7) | <1 | <1 | 2 | 16 | 2 |
| 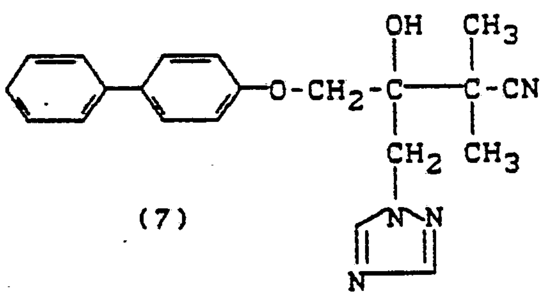 (7) | | | | | |

*) minimale Hemmkonzentration

Tabelle A: (Fortsetzung)

Antimykotische in vitro-Wirksamkeit

| Wirkstoff | MHK*)-Werte in µg/ml Nährmedium Tricho-phyton mentagr. | Micro-sporum canis | Candida albi-cans | Toru-lopsis gla-brata | Asper-gillus fumi-gatus |
|---|---|---|---|---|---|
| (10) | 2 | - | 8 | 16 | 16 |

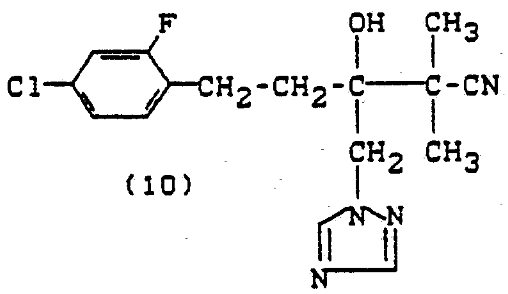

(10)

*) minimale Hemmkonzentration

Beispiel B

Antimykotische in-vivo-Wirksamkeit (oral) bei Mäusecandidose

Versuchsbeschreibung :

Mäuse vom Typ SPF-CF₁ werden intravenös mit 1 - 2 x $10^6$ logarithmisch wachsenden Candida-Zellen, die in physiologischer Kochsalzlösung suspendiert wurden, infiziert. Eine Stunde vor und sieben Stunden nach der Infektion werden die Tiere mit jeweils 25 - 100 mg/kg Körpergewicht der Präparate oral behandelt.

Ergebnis:

Unbehandelte Tiere starben 3 bis 6 Tage post infektionem. Die Überlebensrate am 6.Tag post infektionem betrug bei unbehandelten Kontrolltieren etwa 5 %.

In diesem Test zeigt z.B. die erfindungsgemäß verwendbare Verbindung (8) eine bessere Wirkung als die aus dem Stand der Technik bekannten Verbindungen (A) und (B).

Tabelle B

Antimykotische in-vivo-Wirkung (oral) bei Mäuse-Candidose

| Wirkstoff | Wirkung |
|---|---|
| (A) 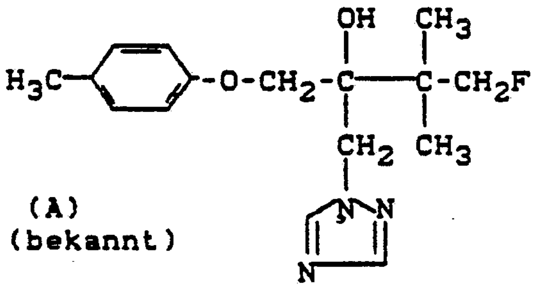 (A) (bekannt) | k.W. |
| (B) 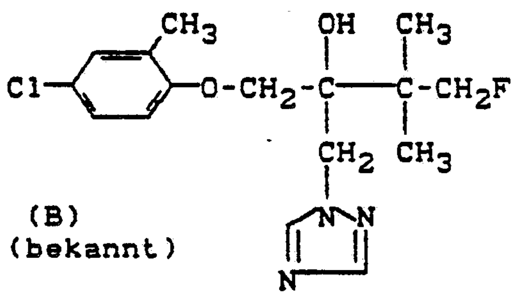 (B) (bekannt) | + |
| (8) 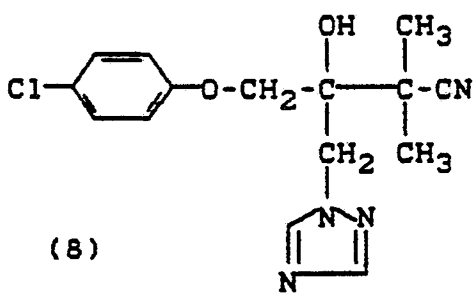 (8) | ++++ |

Zeichenerklärung:

| | | |
|---|---|---|
| +++++ = | sehr gute Wirkung | = 90 % Überlebende am 6.Tag.p.i. |
| ++++ = | gute Wirkung | = 80 % Überlebende am 6.Tag.p.i. |
| +++ = | Wirkung | = 60 % Überlebende am 6.Tag.p.i. |
| ++ = | schwache Wirkung | = 40 % Überlebende am 6.Tag.p.i. |
| + = | Spur Wirkung | = unter 40 % Überlebende am 6. Tag p.i. |
| k.W. | | = kein Unterschied zur unbehandelten Infektionskontrolle |

Beispiel C / Formulierungen

| 1.) Lösung: | | |
|---|---|---|
| Wirkstoff gemäß Formel (I) | : | 10 g |
| Alkohol, rein (96 %ig) | : | 300 g |
| Isopropylmyristat | : | 526 g |
| | | 836 g |

| 2.) Creme: | | |
|---|---|---|
| Wirkstoff gemäß Formel (I) | : | 10 g |
| Arlacel 60 (Sorbitan-monostearat) | : | 20 g |
| Tween 60 (Polyoxyethylen-(20)-sorbitan-monostearat) | : | 15 g |
| Walrat, künstlich (Mischung vom Estern von gesättigten Fettsäuren $C_{14}$-$C_{18}$ und Fettalkoholen $C_{14}$-$C_{18}$) | : | 30 g |
| Lanette O (Gemisch aus Cetyl-Alkohol und Stearyl-Alkohol) | : | 100 g |
| Eutanol G (2-Octyl-dodecanol) | : | 135 g |
| Benzylalkohol | : | 10 g |
| Wasser, entmineralisiert | : | 680 g |
| | | 1000 g |

## Ansprüche

1. Triazolylalkanole der allgemeinen Formel

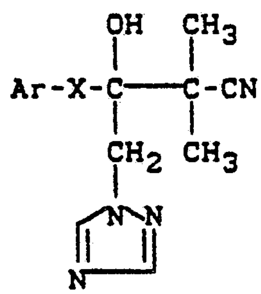

in welcher
Ar für gegebenenfalls substituiertes Aryl steht und
X für eine der Gruppen $-CH_2-$; $-O-CH_2-$; $-S-CH_2-$; $-CH_2-CH_2-$; $-CH=CH-$ oder $-C\equiv C-$ steht,
sowie deren physiologisch verträgliche Säureadditionssalze zur Bekämpfung von Krankheiten.

2. Triazolylalkanole nach Anspruch 1, in denen
Ar für gegebenenfalls einfach oder mehrfach, gleich oder verschieden substituiertes Phenyl steht, wobei als Substituenten infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor und Brom sowie jeweils gegebenenfalls einfach oder mehrfach, gleich oder verschieden durch Halogen substituiertes Phenyl oder Phenoxy und
X für eine der Gruppen $-CH_2-$; $-O-CH_2-$; $-S-CH_2-$; $-CH_2-CH_2-$; $-CH=CH-$ oder $-C\equiv C-$ steht,
sowie deren physiologisch verträgliche Säureadditionssalze zur Bekämpfung von Krankheiten.

3. Triazolylalkanole nach Anspruch 1, in denen
Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n-oder i-Propoxy, Methylthio, Trifluormethyl, Trifluormethoxy, Trifluormethylthio sowie jeweils gegebenenfalls ein- bis dreifach, gleich oder verschieden durch Fluor, Chlor und/oder Brom substituiertes Phenyl oder Phenoxy und
X für eine der Gruppen $-CH_2-$; $-O-CH_2-$; $-S-CH_2-$; $-CH_2-CH_2-$; $-CH=CH-$ oder $-C\equiv C-$ steht,
sowie deren physiologisch verträgliche Säureadditionssalze zur Bekämpfung von Krankheiten.

4. 5-(4-Fluorphenyl)-3-hydroxy-2-methyl-3-(1,2,4-triazolyl-1-ylmethyl)-pentan-2-carbonitril

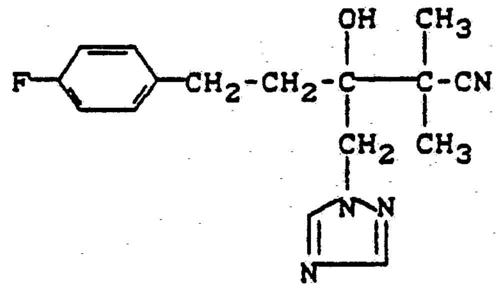

sowie dessen physiologisch verträgliche Säureadditionssalze zur Bekämpfung von Krankheiten.

5. 5-(4-Chlorphenyl)-3-hydroxy-2-methyl-3-(1,2,4-triazolyl-1-ylmethyl)-pentan-2-carbonitril

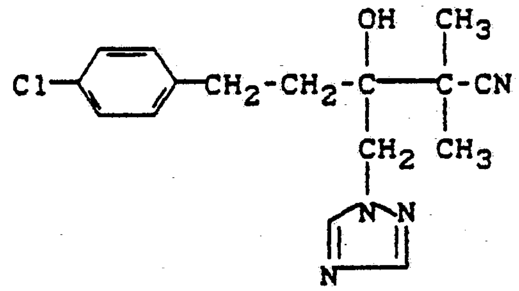

sowie dessen physiologisch verträgliche Säureadditionssalze zur Bekämpfung von Krankheiten.

6. 1-(4-Chlorphenoxy)-2-hydroxy-3-methyl-2-(1,2,4-triazolyl-1-ylmethyl)-butan-3-carbonitril

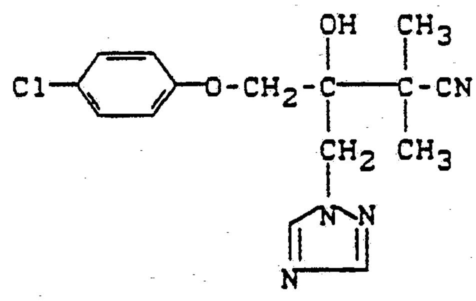

sowie dessen physiologisch verträgliche Säureadditionssalze zur Bekämpfung von Krankheiten.

7. Triazolylalkanole nach Ansprüchen 1-6 zur Bekämpfung von Mykosen.

8. Arzneimittel enthaltend Triazolylalkanole nach Ansprüchen 1-6.

9. Verwendung von Triazolylalkanolen nach Ansprüchen 1-6 bei der Bekämpfung von Krankheiten.

10. Verwendung von Triazolylalkanolen nach Ansprüchen 1-6 bei der Bekämpfung von Mykosen.